# EUROPEAN PATENT SPECIFICATION

(11) **EP 1 940 366 B1**
(45) Date of publication and mention of the grant of the patent: **08.04.2009**
(21) Application number: 07819163.2
(22) Date of filing: 19.10.2007
(51) Int. Cl.: A61K 9/48, A61K 9/66, A61K 31/473

(54) **SOFT CAPSULES COMPRISING PALONOSETRON HYDROCHLORIDE HAVING IMPROVED STABILITY AND BIOAVAILABILITY**
WEICHKAPSELN MIT PALONOSETRON-HYDROCHLORID MIT VERBESSERTER STABILITÄT UND BIOVERFÜGBARKEIT
CAPSULES MOLLES COMPRENANT DU CHLORHYDRATE DE PALONOSÉTRON AYANT UNE STABILITÉ ET UNE BIODISPONIBILITÉ AMÉLIORÉES

(30) Priority: 24.10.2006 US 854342 P
(43) Date of publication of application: 09.07.2008
(73) Proprietor: Helsinn Healthcare S.A., 6915 Pambio-Noranco (CH)
(72) Inventor: BONADEO, Daniele, 21030 Casalzuigno, Varese (IT); CALDERARI, Giorgio, 6862 Rancate (CH); BRAGLIA, Enrico, 6912 Lugano-Pazzallo (CH); BRAGLIA, Riccardo, 6912 Lugano-Pazzallo (CH)
(74) Representative: Leissler-Gerstl, Gabriele
(86) International application number: PCT/EP2007/009098
(87) International publication number: WO 2008/049552

(56) References cited:
- WO-A-02/053131
- WO-A-20/04045615
- WO-A-20/05065652

## Description

### FIELD OF THE INVENTION

The present invention relates to palonosetron, and especially to solid oral dosage forms of palonosetron hydrochloride that meet demanding shelf stability requirements.

### BACKGROUND OF THE INVENTION

The nausea and emetogenic side effects of anti-cancer chemotherapy and radiotherapy are a widespread and longstanding problem. Perhaps less well known but no less important are post-operative nausea and emesis, which may have physiological mechanisms related to the effects seen for chemotherapy. Palonosetron hydrochloride has recently emerged as a highly efficacious anti-nauseant and anti-emetic for use with emetogenic anti-cancer chemotherapies. (Macciocchi, A., et al., "A Phase II dose-ranging study to assesses single intravenous doses of palonosetron for the prevention of highly emetogenic chemotherapy-induced nausea and vomiting," Proc. Am. Soc. Clin. Oncol., 2002; Abstract 1480. Palonosetron also prevents postoperative nausea and vomiting. (Chelly, J., et al., "Oral RS-25259 prevents postoperative nausea and vomiting following laparoscopic surgery," Anesthesiol., 85(Suppl. 21):abstract no 3A (1996)). Methods of treating chemotherapy induced nausea and vomiting (CINV) and radiation induced nausea and vomiting (RINV) with palonosetron are described in PCT publication WO 2004/045615 from Helsinn Healthcare SA. Methods of treating post-operative nausea and vomiting (PONV) with palonosetron are described in PCT publication 2004/073714, also from Helsinn Healthcare SA.

Palonosetron is selective, showing a high affinity as an antagonist for the 5-hydroxyltryptamine 3 receptor precursor (5-HT₃ receptor), and showing a low affinity for other receptors such as dopamine receptors (Wong, E.H.F., et al., "The interaction of RS 25259-197, a potent and selective antagonist, with 5-HT3 receptors, in vitro," Br. J Pharmacol., 114:851-859 (1995); Eglen, R.M., et al., "Pharmacological characterization of RS 25259-197, a potent and selective antagonist, with 5-HT3 receptors, in vivo," Br. J Pharmacol., 114:860-866 (1995)). Palonosetron is a synthetic compound existing as a single isomer, and is administered as the hydrochloride salt, as represented in the following structure: 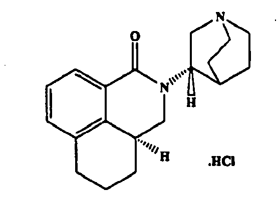 The official chemical name for the drug is (3aS)-2-[(S)-1-Azabicyclo [2.2.2]oct-3-yl]-2,3,3a,4,5,6-hexahydro-1-oxo-1 Hbenz[de] isoquinoline hydrochloride (CAS No. 119904-90-4); its empirical formula is C₁₉H₂₄N₂O·HCl, and its molecular weight is 332.87. Methods of synthesizing the compound are described in U.S. Patent Nos. 5,202,333 and 5,510,486.

Palonosetron hydrochloride is sold as a sterile injectable liquid in the United States as ALOXI^{®} by MGI Pharma and Helsinn Healthcare SA. The intravenous liquid is clear, colorless, non-pyrogenic, in an isotonic, buffered solution. A stable isotonic solution of palonosetron for injection is described in Helsinn's PCT publication WO 2004/067005.

Despite the numerous clinical benefits and advantages of this intravenous formulation, it is generally recognized that injection drug delivery systems present special problems with respect to storage life and stability of the active agent. They are also inconvenient when self administered, and have increased risk of contamination and human error. Thus an oral delivery option for palonosetron, especially in solid form, would be particularly attractive. Methods for improving the stability and shelf-life of palonosetron formulations would also be desirable.

### SUMMARY OF THE INVENTION

Soft-gel capsules of palonosetron have been developed that exhibit excellent bioavailability when orally ingested, and stability when stored for prolonged periods of time. The outer shell for the capsule is gelatin based, and the inner fill for the capsule is a continuous lipophilic inner phase that contains palonosetron dissolved in an aqueous component, miscibilized or homogenized in the lipophilic phase by minimal quantities of a surfactant. The formulation represents an elegant solution to the tension commonly observed between:
- aqueous fills and gelatin stability;
- surfactant and palonosetron degradation; and
- palonosetron stability and palonosetron concentration

In a first principal embodiment, therefore, the invention provides a soft gelatin capsule for oral administration comprising: (a) a soft gelatin outer shell having an oxygen permeability of less than about 1.0 x 10⁻³ ml·cm/(cm²·24 hr. atm); and (b) a lipophilic liquid inner fill composition comprising: (i) greater than about 50wt.% of one or more lipophilic components; (ii) from about 1 to about 20 wt.% of water miscibilized or homogenized in said one or more lipophilic components; (iii) from about 0.05 to about 2.0 mg. of palonosetron as palonosetron hydrochloride solubilized or dispersed in said water; and (iv) from about 0.5 to about 5 wt.% of a surfactant.

Formulations and methods of manufacture have also been developed that can be defined by the amount or concentration of palonosetron in the dosage form, and the degradation byproducts within the dosage form. One such degradation by product is an oxygen mediated degradation product, and is referred to herein as "Cpd1."

Dosage forms of palonosetron, including methods of manufacture, have also been developed with enhanced stability due to their protection from oxygen and oxygen mediated degradation. Based on these discoveries and developments, dosage forms have been developed that can be defined by one or more of the following physical features:
- a shell or coating that is substantially impermeable to oxygen;
- the use of a liquid filling within a capsule shell, preferably containing water;
- a minimal oxygen content in the liquid filling;
- chemical means for preventing oxidative degradation;
- moisture resistant packaging that is resistant to oxygen permeation; and/or
- use of an oxygen-depleted environment while manufacturing the dosage form.

These dosage forms have excellent stability over prolonged periods of time, excellent resistance to oxidative degradation, and excellent bioavailability when orally ingested. These dosage forms can be used in the treatment of any disease for which palonosetron has clinical utility, but they are preferably used for the treatment of emesis.

In a second principal embodiment, therefore, the invention provides a capsule dosage form for oral administration comprising: (a) an outer shell having a oxygen permeability of less than about 1.0 x 10⁻³ ml·cm/(cm²·24 hr. atm); and (b) an inner fill composition comprising: from about 0.05 to about 2.0 mg. of palonosetron as palonosetron hydrochloride, wherein said palonosetron comprises Cpd1 in an amount of less than 1.0 wt.%; wherein no more than 5.0 wt.% of said palonosetron hydrochloride degrades when said dosage form is stored three months or greater at 40°C and 75% RH.

Of course, the invention could be practiced using dosage forms other than capsules, and in another embodiment the invention provides a solid oral dosage form comprising: (a) an outer shell or coating having a oxygen permeability of less than about 1.0 x 10⁻³ ml·cm/(cm²·24 hr. atm); and (b) an inner fill composition comprising: from about 0.05 to about 2.0 mg. of palonosetron as palonosetron hydrochloride, wherein said palonosetron comprises Cpd1 in an amount of less than 1.0 wt.%; wherein no more than 5.0 wt% of said palonosetron hydrochloride degrades when said dosage form is stored three months or greater at 40°C and 75% RH.

Methods have also been developed for manufacturing palonosetron dosage forms that have reduced quantities of impurities and oxygen mediated degradation products, and to palonosetron dosage forms manufactured by these methods. Thus, in still another embodiment the invention provides a method for manufacturing a batch of palonosetron dosage forms having reduced quantities of impurities and oxygen mediated degradation products comprising (a) mixing palonosetron hydrochloride and one or more pharmaceutically acceptable excipients to form a mixture; (b) processing said mixture into a plurality of final dosage forms; and (c) testing one or more of said final dosage forms for Cpd1. This method can be practiced with any dosage form, including a capsule, gel-cap or liquid filled ampoule.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### IN THE FIGURES

Figure 1 plots pharmacokinetics observed in human patients from a bioequivalence study, wherein b1 represents treatment by clinical Formulation A, b2 represents treatment by commercial Formulation B, and b3 represented treatment by Aloxi^{®} i.v.
Figure 2 plots pharmacokinetics observed in human patients from a bioequivalence study, wherein b 1 represents clinical formulation A, and b2 represents commercial formulation B.

Both figures report arithmetic mean plasma concentrations of palonosetron (ng/ml) versus time (H) on a linear scale (n=33).

### DETAILED DESCRIPTION OF THE INVENTION

The present invention may be understood more readily by reference to the following detailed description of preferred embodiments of the invention and the Examples included therein.

### Definitions and Use of Terms

As used in this specification and in the claims which follow, the singular forms "a," "an" and "the" include plural referents unless the context clearly dictates otherwise. Thus, for example, reference to "an ingredient" includes mixtures of ingredients, reference to "an active pharmaceutical agent" includes more than one active pharmaceutical agent, and the like.

"Treating" or "treatment" of a disease includes (1) preventing the disease from occurring in an animal that may be predisposed to the disease but does not yet experience or display symptoms of the disease, (2) inhibiting the disease, i.e. arresting its development, or (3) relieving the disease, i.e. causing regression of the disease.

As used herein, an ambient environment refers to the environment immediately surrounding an element or process, typically a gaseous environment, with which the element or process is in contact and communication.

"Emesis," for the purposes of this application, will have a meaning that is broader than the normal, dictionary definition and includes not only vomiting, but also nausea and retching.

"Moderately emetogenic chemotherapy" refers to chemotherapy in which the emetogenic potential is comparable or equivalent to the emetogenic potential of carboplatin, cisplatin ≤ 50 mg/m², cyclophosphamide < 1500 mg/m², doxorubicin > 25 mg/ms, epirubicin, irinotecan, or methotrexate > 250 mg/m².

"Highly emetogenic chemotherapy" refers to chemotherapy in which the emetogenic potential is comparable or equivalent to the emetogenic potential of cisplatin ≥ 60 mg/m², cyclophosphamide > 1500 mg/m², or dacarbazine.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Therapeutically effective amount" means that amount which, when administered to an animal for treating a disease, is sufficient to effect such treatment for the disease.

A "de minimis" quantity of oxygen refers to an amount of oxygen that allows no more than about 0.5,1.0,1.5,2.0,2.5, or 3.0 wt.% of said palonosetron to degrade (preferably defined by degradation to Cpd1) when stored at room temperature under ambient conditions for six, twelve, eighteen, twenty-four, thirty or thirty-six months.

Shelf stability, for purposes of this invention, is measured by storing the dosage form in its packaging at 40 °C, at a relative humidity of 75%, or under ambient conditions, for three, six, twelve, eighteen, twenty-four, thirty or thirty-six months. A stable formulation is one in which no more than about 0.5, 1.0, 1.5, 2.0, 2.5, 3.0, or 5.0 wt% of the palonosetron in the dosage form degrades (preferably defined by degradation to one or more of the degradation products described herein).

When ranges are given by specifying the lower end of a range separately from the upper end of the range, it will be understood that the range can be defined by selectively combining any one of the lower end variables with any one of the upper end variables that is mathematically possible.

When used herein the term "about" or "ca." will compensate for variability allowed for in the pharmaceutical industry and inherent in pharmaceutical products, such as differences in product strength due to manufacturing variation and time-induced product degradation. The term allows for any variation which in the practice of pharmaceuticals would allow the product being evaluated to be considered bioequivalent to the recited strength of a claimed product.

The term "absolute bioavailability" refers to the availability of the active drug in systemic circulation after non-intravenous administration (i.e., after oral, rectal, transdermal, subcutaneous administration). In order to determine absolute bioavailability of a drug, a pharmacokinetic study must be done to obtain a plasma drug concentration versus time plot for the drug after both intravenous (IV) and non-intravenous administration. The absolute bioavailability is the dose-corrected area under curve (AUC) non-intravenous divided by AUC intravenous. A formulation is said to be bioequivalent in terms of absolute bioavailability to a reference formulation when there is established a 90% confidence interval for AUC_{(0-∞)} which is between 80% and 125%, relative to degree of bioavailability for the reference formulation.

When pharmacokinetic parameters are given herein (i.e. Tₘₐₓ, absolute bioavailability, etc.), it will be understood that they can refer to the mean, median, or individual observed pharmacokinetics, and that mean pharmacokinetics are intended when claimed unless stated to the contrary. The pharmacokinetic parameter will also be understood to be observed in the fasted state, unless otherwise stated.

### Discussion

As mentioned above, the invention provides solid oral dosage forms that have improved stability and resistance to oxidative degradation, based on several formulation techniques, including the use of a coating or shell that is substantially impermeable to oxygen, or the use of a lipophilic liquid filling having water homogenized or miscibilized therein. In a first principal embodiment the invention provides a solid oral dosage form comprising: (a) an outer shell or coating having a oxygen permeability of less than about 1.0 x 10⁻³ ml·cm/(cm²·24 hr. atm); and (b) an inner fill composition comprising: from about 0.05 to about 2.0 mg. of palonosetron as palonosetron hydrochloride, wherein said palonosetron comprises Cpd1 in an amount of less than 1.0 wt.%; wherein said dosage form exhibits shelf stability, preferably defined so that no more than 5.0 wt.% of said palonosetron hydrochloride degrades when said dosage form is stored three months or greater at 40 °C and 75% RH. The invention further provides a method of treating emesis comprising orally administering to a patient suffering from emesis, or at risk for suffering emesis, a dosage form of the present invention.

The invention can be practiced with any type of solid oral dosage form, defined as any dosage form that is administered via the oral route and swallowed including, for example, a capsule or gel-cap (i.e. a liquid filled capsule). In a preferred embodiment the dosage form is a capsule, and in an even more preferred embodiment the dosage form is a liquid filled gel-cap.

Whatever the dosage form, it preferably has an outer shell or coating that has minimal oxygen permeability. In preferred embodiments of the invention, the coating or shell has an oxygen permeability that is less than about 1.0 x 10⁻³, 5.0 x 10⁻⁴, 1.0 x 10⁻⁴, 5.0 x 10⁻⁵, or even 2.0 x 10⁻⁵ ml·cm/(cm²·24 hr. atm).

A preferred dosage form for the present invention is a capsule having an outer shell that dissolves in gastric fluids. A liquid-filled capsule, preferably including water, is especially preferred because of the uniformity of content and dose when working with liquids, and the ability to minimize oxygen exposure while manufacturing the dosage form and storing the dosage form for prolonged periods of time.

Of the available outer shells, a soft outer shell is a preferred shell structure because of its ability to hold liquids and resist oxygen transmission. Preferred materials for the outer "gel-cap" shell include, for example, gelatin, cellulose, starch, or HPMC. In a preferred embodiment, the shell comprises gelatin, and optionally one or more shell excipients selected from glycerin, sorbitol and titanium dioxide.

The liquid composition that fills the capsule is preferably (1) predominantly lipophilic, and (2) present as a continuous liquid phase (i.e. wherein the liquid components are either miscible or completely homogenized/emulsified). A continuous phase is preferred for ease of processing and composition uniformity. The liquid fill includes the excipient base and the active agent evenly distributed throughout the liquid fill. Furthermore, the active agent is preferably dissolved or dispersed as a microemulsion in the excipient base. The total weight of the fill composition may range is preferably greater than about 50, 75, or 100 mg, and is preferably less than about 500, 250, 200, or 150 mg, most preferably from about 100 to about 150mg.

The liquid fill is preferably composed predominantly of one or more lipophilic components in an amount of from about 50 wt.% to about 99 wt.%, preferably from about 75 wt.% to about 98 wt.%. Preferred lipophilic components include, for example, mono- and di-glycerides of fatty acids, especially including the mono- and di-glycerides of capryl/capric acid. The liquid fill may also contain glycerin, preferably in an amount of from about 1 to about 15 wt.%, more preferably from about 2 to about 10 wt.%. In one preferred embodiment, both the shell and the inner fill composition comprise glycerin. In another preferred embodiment, the liquid fill comprises 0.25, 0.35 mg. or more of palonosetron as palonosetron hydrochloride (i.e. 0.50 or 0.75 mg.); solubilized in a solubilizing effective amount of a liquid comprising a lipophilic excipient and water.

The fill composition may comprise various means to facilitate the transition of palonosetron from the dosage form to the gastrointestinal fluids of the GI tract, so that the palonosetron may be more readily absorbed into the bloodstream. For example, the liquid fill composition may contain a surfactant, optimally in an amount of from about 0.1 wt.% to about 6 wt.%, from about 0.5 wt.% to about 5 wt.%, or from about 1.0 wt.% to about 3.0 wt.%. The liquid fill composition preferably comprises greater than 0.1, 0.5, or 1.0 wt.% of surfactant, and less than 10, 8, 5, 4, or even 4 wt% of surfactant. A particularly preferred surfactant is polyglyceryl oleate.

Alternatively or in addition, the transitioning means for a liquid filled capsule may comprise water that forms a single phase or microemulsion with the other liquid ingredients in the excipient base. The liquid fill composition preferably comprises from about 0.05 wt.% to about 30 wt% water, from about 1 wt.% to about 20 wt.% water, or from about 2 wt.% to about 10 wt.% water. The liquid fill preferably comprises greater than 0.1, 0.5 or 1.0 wt.% water, and less than 20,15,10,8 or 5 wt% water.

Still further, the excipient base may contain one or more chemical agents to prevent oxygen mediated degradation of the palonosetron in the dosage form. For example, the excipient base may contain a chelating agent such as ethylenediamine tetraacetic acid (EDTA), an antioxidant such as butylated hydroxyanisole (BHA), or a reducing agent, in an amount ranging from about 0.005 wt% to about 2.0 wt.%, more preferably from about 0.01 wt.% to about 1.0 wt.% or from about 0.05 wt.% to about 0.5 wt.%. In a preferred embodiment the excipient base contains an antioxidant.

The active agent, which is preferably palonosetron hydrochloride, is preferably present in the fill composition in an amount ranging from about 0.01 to about 10.0 wt.%, from about 0.05 to about 5.0 wt.%, or from about 0.1 wt% to about 2.0 wt.%. Alternatively, particularly stable formulations have been found where the concentration of palonosetron exceeds 0.3%, preferably at a concentration no greater than about 1 wt.%.

A particularly important feature of the inner fill composition, which is preferred in any of the embodiments of this invention, regardless of dosage form or fill type or method of manufacture, is the minimal content of oxygen. In a preferred embodiment, the inner fill composition comprises oxygen in an amount that degrades no more than about 3.0 wt.%, 2.5 wt.%, 2.0 wt.%, 1.5 wt.%, 1.0 wt.%, or 0.5 wt.%, of said palonosetron, when the dosage form is stored under shelf stability testing regimens, for example for three months at 40°C and 75% RH. This amount is preferably measured by the amount of Cpd1 in the composition.

Another important feature of the formulations of the present invention is their pharmacokinetics. It has been determined that the dosage forms of the current invention have an absolute bioavailability of approximately 100%, within the limits of bioequivalence. Thus, for example, whereas a 0.75 mg injection of palonosetron yields a mean AUC_{(0-∞)} of ca. 58285 (ng· hr/L), a 0.75 mg gel cap yields a mean AUC_{(0-∞)} of ca. 57403 (ng· hr/L). In contrast, the mean Cₘₐₓ for a 0.75 mg gel cap is about 1224 ng/L, whereas a 0.75 mg. injection yields a mean Cₘₐₓ of about 1665 ng/L. A 0.50 mg gel cap has been shown to yield a mean AUC_{(0-∞)} of ca. 38176 (ng· hr/L), and a mean Cₘₐₓ of about 810 ng/L, thereby demonstrating dose proportionate pharmacokinetics.

In various embodiments, therefore, the dosage form of the present invention yields greater than 90, 95, or even 98 % absolute bioavailability as an arithmetic mean, again within the limits of bioequivalence. Alternatively or in addition, a 50 mg gel cap yields a mean Cₘₐₓ of from about 700 to about 950 ng/ml, or from about 750 to about 875 ng/ml. In a most preferred embodiment, a 50 mg gel gap yields a Cₘₐₓ of from 800 to 820 ng/L, preferably within the limits of bioequivalence. Because the dosage forms of the present invention demonstrate dose proportionate pharmacokinetics, it will be understood that these Cₘₐₓ values can be standardized based on the strength of the dosage form, and that Cₘₐₓ values can be assigned to alternative strengths based upon such standardization.

Yet another important feature of the dosage forms of the present invention, which also is preferred in any of the embodiments of the present invention, pertains to the dissolution of the dosage form, and in a preferred embodiment no less than about 75% of the palonosetron in the dosage form dissolves in 30 or 45 minutes when tested in a type II paddle dissolution apparatus according to the U.S. Pharmacopeia, at 75 rpm and 37 °C, in 500 ml. of 0.01N HCl.

Still another feature of the dosage forms of the present invention, which is also preferred in any of the embodiments of the present invention, regardless of dosage form or fill type or method of manufacture, is that the dosage form experiences no more than 5 wt.%, 3 wt.%, or 2 wt.% degradation of the palonosetron when the dosage form in its moisture resistant packaging is exposed to an environment of 25 °C and 60% RH, or 40 °C and 75% RH, for periods equal to or exceeding 3 months, six months, 9 months or even one year.

### Palonosetron Hydrochloride and Related Compounds

The palonosetron used in the present invention can be palonosetron as a base or pharmaceutically acceptable salt, but is preferably palonosetron hydrochloride. In addition, the palonosetron is preferably present in an amount ranging from about 0.02 mg. to about 10 mg. per dosage form, more preferably from about 0.05 or 0.15 to about 2 mg. per dosage form, and still more preferably from about 0.2 to about 1.0 mg. per dosage form, based on the weight of the base when present as a pharmaceutically acceptable salt. Particularly preferred doses are 0.25 mg, 0.50, and 0.75 mg. of palonosetron or salt thereof, based on the weight of the base. Particularly stable formulations have been found by using palonosetron amounts in liquid gel-caps of greater than about 0.25, 0.35 or 0.45 mg., preferably less than about 2.0 mg.

The palonosetron hydrochloride used to make the dosage form, or contained in the final dosage form, may also be characterized by the presence of various palonosetron related compounds, including compounds Cpd3, Cpd2, and/or Cpd1 as described by the following chemical structures:

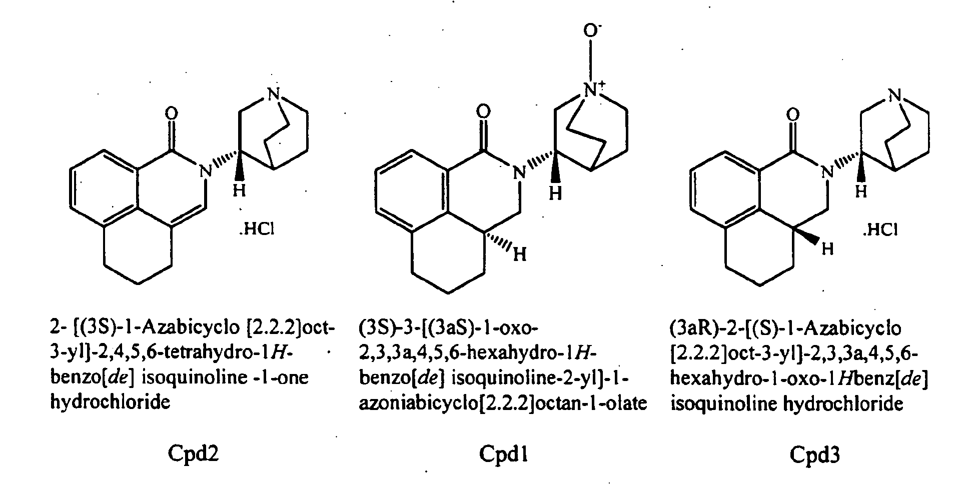

Compounds Cpd2 and Cpd3 are typically present, on an individual or combined basis relative to the palonosetron hydrochloride, in amounts of less that 1.0 wt.%, 0.75 wt.% or 0.5 wt.%, and/or greater than about 0.05 wt.%, 0.075 wt.% or 0.1 wt.%. Cpd2 and Cpd3 can be measured in the dosage form or in the palonosetron raw material used to make the dosage form. Compound Cpd1 is typically present, on an individual basis relative to the palonosetron hydrochloride, in an amount greater than about 0.05 wt.%, 0.1 wt.% or 0.2 wt.%, and/or less than about 3.0 wt.%, 2.5 wt.%, 2.0 wt.%, 1.5 wt.%, 1.0 wt.%, or 0.5 wt.%. Cpd1 is preferably measured in the dosage form since it is a measure of oxygen mediated degradation. In one preferred embodiment, the dosage forms are defined by a stability in which no more than about 5.0 wt.%, 4.0 wt.%, 3.0 wt.%, 2.5 wt.%, 2.0 wt.%, 1.5 wt.%, 1.0 wt.%, or 0.5 wt.% of compound Cpd1, are formed when the dosage form in its moisture resistant packaging is exposed to an environment of 25 °C and 60% RH, or 40 °C and 75% RH, for periods equal to or exceeding 3 months, 6 months, 9 months or even one year.

Therefore, in another embodiment the invention provides a solid oral dosage form comprising: (a) from about 0.05 to about 2.0 mg of palonosetron or a pharmaceutically acceptable salt thereof; (b) one or more pharmaceutically acceptable excipients; (c) Cpd1 in an amount less than 3.0 wt.% based on the weight of the palonosetron. In another embodiment the invention provides a solid oral dosage form comprising: (a) from about 0.05 to about 2.0 mg. of palonosetron or a pharmaceutically acceptable salt thereof; (b) one or more pharmaceutically acceptable excipients; (c) Cpd2 or Cpd3, in an amount less than 1.0 wt.%, based on the weight of the palonosetron or pharmaceutically acceptable salt thereof. In either of these embodiments, the dosage form may optionally comprise means for preventing oxygen mediated degradation of said palonosetron.

Other palonosetron related compounds that can be present in the compositions include Cpd4, Cpd5, Cpd6 and Cpd7, as depicted below:

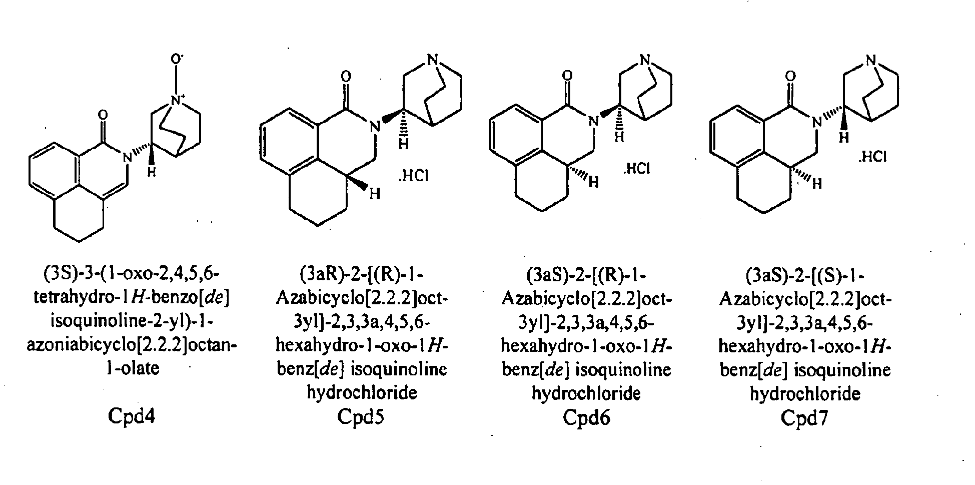

### Methods of Making

The invention also provides methods of making palonosetron dosage forms. Thus, in still another embodiment the invention provides a method for manufacturing a batch of palonosetron dosage forms having reduced quantities of impurities and oxygen mediated degradation products comprising (a) mixing palonosetron hydrochloride and one or more pharmaceutically acceptable excipients to form a mixture; (b) processing said mixture into a plurality of final dosage forms; and (c) testing one or more of said final dosage forms for one or more palonosetron related compounds selected from Cpd2, Cpd1, and Cpd3. "Processing" refers to the steps used to prepare a pharmaceutical formulation and final dosage form from a defined set of ingredients, and excludes the processes of chemically synthesizing the ingredients used in the formulation. This embodiment extends to all dosage forms of palonosetron, including single unit dose ampoules of palonosetron filled, for example, with a sterile injectable liquid. Thus, for example, the invention may be extended to methods for filling unit dose ampoules or containers with sterile injectable solutions of palonosetron, preferably in aqueous media, and preferably formulated as described in WO 2004/067005 of Calderari et al. In this context, an "ampoule" means a small sealed container of medication that is used one time only, and includes breakable and non-breakable glass ampoules, breakable plastic ampoules, miniature screw-top jars, and any other type of container of a size capable of holding only one unit dose of palonosetron (typically about 5 mls.).

Another embodiment captures the balance achieved by the formulations of the present invention, relative to bioavailability and stability, and in this embodiment the invention provides a method of optimizing the bioavailability and stability of palonosetron in a palonosetron gelatin capsule comprising: (a) providing a soft gelatin outer shell having an oxygen permeability of less than about 1.0 x 10⁻³ ml·cm/(cm²·24 hr. atm); and (b) preparing a fill composition by steps comprising: (i) providing from about 0.05 to about 2.0 mg. of palonosetron as palonosetron hydrochloride, wherein said palonosetron comprises Cpd1 in an amount of less than 1.0 wt.% based on the weight of said palonosetron; (ii) dissolving or dispersing said palonosetron in water to form an aqueous premix; (iii) mixing said aqueous premix with one or more lipophilic excipients, at a weight ratio of aqueous premix to lipophilic excipients of less than 50:50. 40:60, 30:70, or 20:80, to form a miscible or homogenous lipophilic fill composition; (iv) mixing a surfactant with said water, said aqueous premix, or said fill composition; and (v) balancing the quantities of surfactant and water in said fill composition to facilitate the bioavailability ofpalonosetron from said gelatin capsule when orally ingested, and to minimize the degree of palonosetron degradation; and (c) filling said outer shell with said fill composition.

Still another method of the present invention comprises a method of packaging a palonosetron dosage form comprising: (a) providing an empty shell; and (b) filling said shell container with a fill composition in an oxygen depleted ambient environment, wherein said fill composition comprises: (i) a defined amount of an active ingredient composition comprising palonosetron or a pharmaceutically acceptable salt thereof; and (ii) a pharmaceutically acceptable excipient. An "oxygen depleted environment" is preferably one defined by an oxygen content of less than about 10% oxygen, 5% oxygen, or even 1% or 0.1 % oxygen (on a weight or volume basis). In an even more preferred embodiment, the methods of making or packaging the dosage forms of the present invention are performed under a nitrogen blanket or purge, in a nitrogen rich environment comprising greater than about 90%, 95%, or 98% nitrogen (on a weight or volume basis).

In another particular embodiment, the method is defined by the variability of active ingredient among dosage forms, in which there is provided a method of making a plurality of solid oral dosage forms comprising: (a) providing a capsule shell; (b) filling said shell with a fill composition comprising: (i) a defined amount of palonosetron or a pharmaceutically acceptable salt thereof; and (ii) a pharmaceutically acceptable excipient; and (c) repeating steps (a) and (b) one or more additional times, wherein said defined amount has a capsule to capsule variability of less than about 3, 2, 1, 0.5 or 0.1 wt.%.

In any of the foregoing embodiments, the method of making may also further comprise packaging said dosage form or plurality of dosage forms in a moisture resistant sealed container. The material used to form the moisture resistant sealed container preferably has an oxygen permeability less than about 1.0 x 10⁻², 1.0x 10⁻³, 1.0x 10⁻⁴ , or even 5.0 x 10⁻⁵ ml·cm/(cm²·24 hr. atm). Alternatively or in addition, the packaging can be characterized as a "tight container" under standards described in USP <671> (i.e. not more than one of ten test containers exceeds 100 mg. per day per L in moisture permeability, and none exceeds 200 mg. per day per ml.). Still further, the container can be defined by the amount of moisture that it allows the dosage forms of the invention to absorb during storage. For example, in various preferred embodiments, the container prevents said doses from absorbing more than 1.0, 0.1 or even 0.05 wt.% moisture in three months when stored at 40 °C and 75% relative humidity. Blister packaging is a particularly preferred mode of packaging.

### Soft Gelatin Capsules

The liquid core pharmaceutical compositions of the present invention are encapsulated in a soft gelatin shell described below. Gelatin is a preferred component of the soft gelatin shells of the instant invention. The starting gelatin material may be obtained by the partial hydrolysis of collagenous material, such as the skin, white connective tissues, or bones of animals. Gelatin material can be classified as Type A gelatin, which is obtained from the acid-processing of porcine skins and exhibits an isoelectric point between pH 7 and pH 9; and Type B gelatin, which is obtained from the alkaline-processing of bone and animal (bovine) skins and exhibits an isoelectric point between pH 4.7 and pH 5.2. Blends of Type A and Type B gelatins can be used to obtain a gelatin with the requisite viscosity and bloom strength characteristics for capsule manufacture. Gelatin suitable for capsule manufacture is commercially available from the Sigma Chemical Company, St. Louis, Mo. For a general description of gelatin and gelatin-based capsules, see Remington's Pharmaceutical Sciences, 16th ed., Mack Publishing Company, Easton, Pa. (1980), page 1245 and pages 1576-1582; and U.S. Pat. No. 4,935,243, to Borkan et at., issued Jun. 19, 1990; these two references being incorporated herein by reference in their entirety.

The soft gelatin shells may comprise from about 20% to about 60% gelatin. The gelatin can be of Type A or Type B, or a mixture thereof with bloom numbers ranging from about 60 to about 300. The soft gelatin shells may also comprise a plasticizer. Useful plasticizers include glycerin, sorbitan, sorbitol, or similar low molecular weight polyols, and mixtures thereof. A preferred plasticizer useful in the present invention is glycerin. The soft gelatin shells of the instant invention may also comprise water. Without being limited by theory, the water is believed to aid in the rapid dissolution or rupture of the soft gelatin shell upon contact with the gastrointestinal fluids encountered in the body.

Soft gelatin capsules and encapsulation methods are described in P. K. Wilkinson et at., "Softgels: Manufacturing Considerations", Drugs and the Pharmaceutical Sciences, 41 (Specialized Drug Delivery Systems), P. Tyle, Ed. (Marcel Dekker, Inc., New York, 1990) pp.409-449; F. S. Horn et at., "Capsules, Soft", Encyclopedia of Pharmaceutical Technology, vol. 2; J. Swarbrick and J. C. Boylan, eds. (Marcel Dekker, Inc., New York, 1990) pp. 269-284; M. S. Patel et at., "Advances in Softgel Formulation Technology", Manufacturing Chemist, vol. 60, no. 7, pp. 26-28 (July 1989); M. S. Patel et al., "Softgel Technology", Manufacturing Chemist, vol. 60, no. 8, pp. 47-49 (August 1989); R. F. Jimerson, "Softgel (Soft Gelatin Capsule) Update", Drug Development and Industrial Pharmacy (Interphex '86 Conference), vol. 12, no. 8 & 9, pp. 1133-1144 (1986); and W. R. Ebert, "Soft Elastic Gelatin Capsules: A Unique Dosage Form", Pharmaceutical Technology, vol. 1, no. 5, pp. 44-50 (1977); these references are incorporated by reference herein in their entirety. The resulting soft gelatin capsule is soluble in water and in gatrointestinal fluids. Upon swallowing the capsule, the gelatin shell rapidly dissolves or ruptures in the gastrointestinal tract thereby introducing the pharmaceutical actives from the liquid core into the body.

### Methods of Treatment

In still further embodiments, the invention provides methods of treating emesis by administering one or more of the dosage forms described herein. The emesis may be acute phase emesis (i.e. emesis experienced within about 24 hours of an emesis inducing event), or delayed emesis (i.e. emesis experienced after the acute phase, but within seven, six, five or four days of an emesis inducing event). The emesis may constitute chemotherapy induced nausea and vomiting ("CINV"), from moderately or highly emetogenic chemotherapy, radiation therapy induced nausea and vomiting ("RINV"), or post-operative nausea and vomiting ("PONV").

### Bioequivalence Testing

When a product is said to exhibit a particular pharmacokinetic parameter "within the limits of bioequivalence," it will be understood that the product is bioequivalent to a test drug employing the bioequivalence testing specified herein. Bioequivalence testing typically requires an in vivo test in humans in which the concentration of the active ingredient or active moiety, and, when appropriate, its active metabolite(s), in whole blood, plasma, serum, or other appropriate biological fluid is measured as a function of time. Defined as relative bioavailability ("BA"), bioequivalence ("BE") involves a comparison between a test and reference drug product. Although BA and BE are closely related, BE comparisons normally rely on (1) a criterion, (2) a confidence interval for the criterion, and (3) a predetermined BE limit.

A standard in vivo BE study design is based on the administration of either single or multiple doses of the test and reference products to healthy subjects on separate occasions, with random assignment to the two possible sequences of drug product administration. Statistical analysis for pharmacokinetic measures, such as area under the curve (AUC) and peak concentration (Cₘₐₓ), is preferably based on the so-called "two one-sided tests procedure" to determine whether the average values for the pharmacokinetic measures determined after administration of the test and reference products are comparable. This approach is termed average bioequivalence and involves the calculation of a 90% confidence interval for the ratio of the averages (population geometric means) of the measures for the test and reference products. To establish BE, the calculated confidence interval should fall within a BE limit, i.e. 80-125% for the ratio of the product averages. Thus, for example, bioequivalence is said to be established under a given set of circumstances by a 90% confidence interval for AUC which is between 80% and 125%, and a 90% confidence interval for Cₘₐₓ which is between 80% and 125%.

Further detail regarding BE procedures can be found in FDA's July 1992 Guidance Document entitled "Statistical Procedures for Bioequivalence Studies Using a Standard Two-Treatment Crossover Design," the contents of which are incorporated herein by reference.

### EXAMPLES

The following examples are put forth so as to provide those of ordinary skill in the art with a complete disclosure and description of how the compounds claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C or is at room temperature, and pressure is at or near atmospheric

### Example 1 - Representative Gel-cap Formulation

Table 1 describes representative formulations for a gel-cap solid oral dosage form containing 0.25, 0.50 and 0.75 mg. of palonosetron.

**Table 1. Representative Gel-cap Formulation**

| Names of Ingredients | Formula (mg per capsule) | | |
|---|---|---|---|
| | 0.25 mg | 0.50 mg | 0.75 mg |
| *Active drug substance* Palonosetron HCl | 0.28^{a} | 0.56^{b} | 0.84^{c} |
| | | | |
| *Excipients* Purified water | 5.57 | 5.57 | 5.57 |
| | | | |
| Glycerin, anhydrous | 6.40 | 6.40 | 6.40 |
| | | | |
| Butylated hydroxyanisole (BHA) | 0.13 | 0.13 | 0.13 |
| Polyglyceryl oleate (Plurol Oleique CC 497) | 6.65* | 6.65* | 6.65* |
| | (1.66)** | (1.66)** | (1.66)** |
| Mono- and di-glycerides of Capryl/Capric | 113.97* | 113.69* | 11.3.41* |
| | | | |
| Acid (Capmul MCM) | (118.96)** | (118.68)** | (118.40)** |
| | | | |
| Nitrogen | --- | --- | --- |
| Theoretical fill weight | 133.00 mg | 133.00 mg | 133.00 mg |
| Gelatin Capsule Shell, #3, oval (Cardinal Health) | 1 capsule | 1 capsule | 1 capsule |

| | | | |
|---|---|---|---|
| ^{a} corresponding to 0.25 mg free base ^{b} corresponding to 0.50 mg free base ^{c} corresponding to 0.75 mg free base * Formulation A (clinical batch) ** Formulation B (commercial batch) | | | |

### Example 2 - Manufacturing Protocol

The compounding process involves the formulation of two separate mixes, the side mix containing the active ingredient, glycerin and water, and the main mix containing the remaining excipients. The process starts with the two separate mixes which are later combined to comprise the final fill solution for encapsulation. The fill solution is blanketed with nitrogen during the compounding and encapsulation phases.

### Example 3 - Representative Dissolution Test Protocol

An exemplary dissolution method for Palonosetron Oral Capsules, 0.25 mg, 0.50 mg, and 0.75 mg uses USP Apparatus 2 (paddles) at 75 rpm in 500 mL of 0.01N HCl with a dissolution temperature of 37.0 ± 0.5°C. The acceptance criterion is "Not less than 75 % at 45 minutes".

Six softgel-capsules are individually weighed. Softgel-capsules are placed in each vessel, and sampled at 15, 30, 45, and 60 minutes. Sampling at 15, 30, 60 minutes is for information only. Sample solutions are withdrawn and filtered through online filters into test tubes or HPLC vials. The samples are analyzed using a HPLC system with UV detector.

**Table 2. Dissolution Conditions**

| | |
|---|---|
| USP Apparatus | 2 Paddles |
| Medium | 0.01N HCl, 500 mL |
| Temperature | 37 ± 0.5°C |
| Rotation Speed | 75 rpm |
| Sampling Times | 45 minutes 15, 30, and 60 minutes (for information only) |
| Sampling Volume | 3 mL (or 1 - 1.5 mL when collected directly into HPLC vials) |
| Volume | 500 mL dissolution medium |

### Example 4 - Chemical and Physical Stability

Table 3 presents the results of chemical and physical stability testing for the 0.75 mg. palonosetron softgel formulations reported in Example 1, packaged in a 2x5 Blister Unit (Forming: LM 15088, Foil: Reynolds 701).

**Table 3. Chemical and Physical Stability**

| Test | Palonosetron assay | | Palonosetron related substances | | | Dissolution test (% dissolved) | | | |
|---|---|---|---|---|---|---|---|---|---|
| Provisional Specification | 90.0-110.0 % | % vs t0 | Cpd3 | Cpd2 | Cpd1 | 45 min. | 15 min. | 30 min. | 60 min. |
| | | | ≤ 0.50% | ≤ 0.50% | ≤ 3.0% | NLT 75% | For inform. Only | For inform. only | For inform. only |
| Initial values | 97.7 | 100.0 | 0.28 | 0.28 | 0.20 | 98.5 | 70.4 | 97.7 | 98.9 |
| 25°C/60% r.h. | | | | | | | | | |
| 03 months | 97.6 | 99.9 | 0.23 | 0.26 | 0.23 | 99.2 | 97.6 | 99.1 | 98.8 |
| 06 months | 96.5 | 98.8 | 0.26 | 0.27 | 0.46 | 97.6 | 87.4 | 97.2 | 97.7 |
| 09 months | 96.0 | 98.3 | 0.23 | 0.27 | 0.6 | 99.0 | 80.5 | 98.9 | 99.1 |
| 12 months | 93.7 | 95.9 | 0.22 | 0.26 | 0.6 | 96.8 | 81.9 | 96.5 | 96.7 |
| 40°C/75% r.h. | | | | | | | | | |
| 01 month | 97.2 | 99.5 | 0.36 | 0.25 | 0.56 | 99.9 | 92.3 | 100.5 | 100.1 |
| 03 months | 97.5 | 99.8 | 0.24 | 0.26 | 0.65 | 97.3 | 79.9 | 97.0 | 97.5 |
| 06 months | 96.2 | 98.5 | 0.26 | 0.27 | 0.68 | 96.9 | 52.5 | 96.7 | 97.0 |

### Example 5 - Chemical and Physical Stability

Table 4 presents the results of chemical and physical stability testing for the 0.50 mg. palonosetron softgel formulations reported in Example 1, packaged in a 2x5 Blister Unit (forming: LM 15088, Foil: Reynolds 701).

**Table 4. Chemical and Physical Stability**

| Test | Palonosetron assay | | Palonosetron related substances | | | Dissolution test (% dissolved) | | | |
|---|---|---|---|---|---|---|---|---|---|
| Provisional. Specification | 90.0- 110.0 % | % vs t0 | Cpd3 | Cpd2 | Cpd1 | 45 min. | 15 min. | 30 min. | 60 min. |
| | | | ≤ 3.0% | ≤ 0.50% | ≤ 3.0% | NLT 75% | For inform. only | For inform. only | For inform, only |
| Initial values | 97.9 | 100.0 | 0.28 | 0.26 | 0.16 | 98.9 | 69.1 | 99.9 | 99.2 |
| 25°C/60% r.h. | | | | | | | | | |
| 03 months | 97.5 | 99.6 | 0.23 | 0.26 | 0.44 | 100.1 | 86.0 | 100.0 | 100.1 |
| 06 months | 97.2 | 99.3 | 0.27 | 0.28 | 0.37 | 97.6 | 41.4 | 88.3 | 97.6 |
| 09 months | 96.5 | 98.6 | 0.22 | 0.28 | 0.5 | 99.0 | 83.0 | 97.8 | 99.1 |
| 12 months | 94.7 | 96.7 | 0.22 | 0.27 | 0.5 | 96.9 | 85.6 | 96.7 | 97.0 |
| 40°C/75% r.h. | | | | | | | | | |
| 01 month | 97.3 | 99.4 | 0.29 | 0.27 | 0.42 | 102.6 | 65.8 | 101.1 | 102.8 |
| 03 months | 97.3 | 99.4 | 0.24 | 0.26 | 0.55 | 100.0 | 39.8 | 94.2 | 99.0 |
| 06 months | 96.6 | 98.7 | 0.26 | 0.27 | 0.67 | 97.0 | 52.7 | 96.7 | 97.2 |

### Example 6 -- Chemical and Physical Stability

Table 5 presents the results of chemical and physical stability testing for the 0.25 mg. palonosetron softgel formulations reported in Example 1, packaged in a 2x5 Blister Unit (Forming: LM 15088, Foil: Reynolds 701).

**Table 5. Chemical and Physical Stability**

| Test | Palonosetron assay | | Palonosetron related substances | | | Dissolution test (% dissolved) | | | |
|---|---|---|---|---|---|---|---|---|---|
| Provisional Specification | 90.0- 110.0 % | % vs t0 | Cpd3 | Cpd2 | Cpd1 | 45 min. | 15 min. | 30 min. | 60 min. |
| | | | ≤ 0.50% | ≤0.50% | ≤3.0% | NLT 75% | For inform. only | For inform. only | For inform. only |
| Initial values | 97.7 | 100.0 | 0.29 | 0.29 | 0.38 | 97.2 | 61.8 | 97.4 | 97.7 |
| 25°C/60% r.h. | | | | | | | | | |
| 03 months | 97.4 | 99.7 | 0.24 | 0.26 | 0.92 | 98.6 | 98.0 | 99.1 | 99.1 |
| 06 months | 95.9 | 98.2 | 0.28 | 0.27 | 1.10 | 95.2 | 82.3 | 94.8 | 95.7 |
| 09 months | 94.7 | 96.9 | 0.21 | 0.28 | 1.4 | 94.5 | 89.2 | 93.9 | 94.6 |
| 12 months | 94.5 | 96.7 | 0.23 | 0.27 | 1.7 | 96.2 | 97.1 | 96.1 | 96.1 |
| 40°C/75% r.h. | | | | | | | | | |
| 01 month | 96.3 | 98.6 | 0.29 | 0.27 | 1.42 | 99.4 | 88.2 | 99.5 | 97.7 |
| 3 months | 97.4 | 99.7 | 0.24 | 0.25 | 1.85 | 97.4 | 78.7 | 97.6 | 97.5 |
| 6 months | 94.0 | 96.2 | 0.27 | 0.26 | 1.94 | 96.8 | 82.6 | 96.2 | 97.0 |

### Example 7 -- Representative Injectable Formulation

The following Table 6 describes a representative injectable formulation containing palonosetron.

**Table 6. Representative Injectable Formulation**

| Ingredient | mg/mL |
|---|---|
| Palonosetron Hydrochloride | 0.05 (calculated as base) |
| Mannitol | 41.5 |
| EDTA | 0.5 |
| Trisodium citrate | 3.7 |
| Citric acid | 1.56 |
| WFJ | 1.0 |
| Sodium hydroxide solution and/or hydrochloric acid solution | pH 5.0 ± 0.5 |
| Flavoring | q.s. |

### Example 8 -- Identification and Assay of Palonosetron in Palonosetron HCl Softgels by HPLC with UV Detector

### Testing procedure

Prepare Sample and Standard solutions at the Palonosetron HCl nominal concentration of 6.25 µg/mL in HCl 0.01 N.
Filter solution and inject into HPLC system.

| HPLC Condition | |
|---|---|
| Column | C8, 250 mm x 4.6 mm (i.d.) |
| Column Temperature | 30°C |
| Mobile Phase | ACN/H₂O/ TFA, Gradient Elution |
| Flow rate | 1 mL/min |
| Detection | UV at 210 nm |
| Injection Volume. | 20 µL |

### Example 9 -- Determination of Palonosetron Related Compounds in Palonosetron HCl Softgels and the In-Process Assay of the Softgels Fill Solution

### Testing procedure

Prepare Sample and Standard solutions at the Palonosetron HCl nominal concentration of 0.15 mg/mL in Methanol.
Inject solutions directly into HPLC system.

| HPLC Condition | |
|---|---|
| Column | C8, 250 mm x 4.6 mm (i.d.) |
| Column Temperature | 30°C |
| Mobile Phase | ACN/H₂O/ TFA, Gradient Elution |
| Flow rate | 1 mL/min |
| Detection | UV at 210 nm |
| Injection Volume | 10 µL |

### Example 10 -- Determination of Palonosetron Related Compounds in Palonosetron HCl Softgels by Chiral HPLC with UV Detector

### Testing procedure

Prepare Sample solution at the Palonosetron HCl nominal concentration of 0.34 mg/mL in Methanol.
Prepare Cpd2 Standard solution at the nominal concentration of 5.6 µg/mL.
Prepare Resolution solution, in methanol solvent, at the nominal concentration of 8 µg/mL:Cpd2, Cpd4, Cpd5, Cpd6 and Cpd7 concentration approx. 0.4 µg/mL.
Inject solutions directly into HPLC system.

| HPLC Condition | |
|---|---|
| Column | Chiral column |
| Column Temperature | 35°C |
| Mobile Phase | ACN/MeOH/ IPA/AcOH/TEA, Isocratic elution |
| Flow rate | 1 mL/min |
| Detection | UV at 238 nm |
| Injection Volume | 10 µL |

### Example 11 -- Dissolution of Palonosetron HCl Softgels with Assay by HPLC

### Testing procedure

Prepare Standard solutions at the Palonosetron HCl nominal concentration of 1 µg/mL in HCl 0.01 N.
Sample solution: place one softgel-capsule into a vessel containing 500 mL of 0.01 N HCl.
Filter solutions and inject into HPLC system.

| HPLC Condition | |
|---|---|
| Column | C8, 150 mm x 4.6 mm (i.d.) |
| Column Temperature | 30°C |
| Mobile Phase | ACN/H₂O/ TFA, Gradient Elution |
| Flow rate | 1 µL/min |
| Detection | UV at 210 nm |
| Injection Volume | 50 µL |

### Example 12 -- Bioequivalence of 0.75 mg. gel cap and injection dosage forms

Bioequivalence and absolute bioavailability were tested in a single oral dose of two formulations of 0.75 mg palonosetron in healthy volunteers. The study was a three treatment, three period, two sequence cross-over study.

Treatment A represented a single dose of 0.75 mg of palonosetron in the clinical gel-cap formulation described in Table 1.

Treatment B represented a single dose of 0.75 mg of palonosetron in the commercial gel-cap formulation in Table 1.

Treatment IV consisted of three consecutive bolus injections of Aloxi 25 mg.

Pharmacokinetic parameters are reported below in Table 7:

**Table 7**

| **Pharmacokinetic parameter Palonosetron [unit]** | | **0.75 mg Palonosetron oral administration (formulation A)** | **0.75 mg Palonosetron oral administration (formulation B)** | **0.75 mg Palonosetron intravenous administration (3 x 0.25 mg i.v. Aloxi^{®}** |
|---|---|---|---|---|
| | | **N=33** | **N =33** | **N=30** |
| AUC₍₀₋₁₎ [ng·h/L] | Mean (SD) | 53835 (17961) | 55235 (17817) | 53088 (15233) |
| | Geo.Mean (Geo.SD) | 50716 (1.44) | 52536 (1.39) | 50793 (1.37) |
| | Median | 50978 | 53325 | 50984 |
| | Minimum-Maximum | 16971 - 96273 | 20951-111916 | 20609 - 78424 |
| AUC₍₀₋ₓ₎ [ng·h/L) | Mean (SD) | 57403 (17898) | 58285 (18110) | 56480 (15343) |
| | Geo.Mean (Geo.SD) | 54539 (1.40) | 55638 (1.37) | 54324 (1.34) |
| | Median | 54614 | 56802 | 54011 |
| | Minimum-Maximum | 18773 - 100234 | 24473 - 114765 | 24142 - 81547 |
| Cₘₐₓ [ng/L] | Mean (SD) | 1223.985 (348.324) | 1200.620 (324.606) | 1665.314 (527.638) |
| | Geo.Mean (Geo.SD) | 1178.670 (1.32) | 1160.078 (1.31) | 1588.758 (1.37) |
| | Median | 1208.136 | 1133.115 | 1628.480 |
| | Minimum-Maximum | 570.494-2365.980 | 571.922-2130.740 | 890.742-2789.077 |
| tₘₐₓ [h] | Median | 4.520 | 4.530 | 0.250 |
| | Minimum-Maximum | 2.000 - 8.000 | 2.000 - 12.030 | 0.250 - 4.030 |

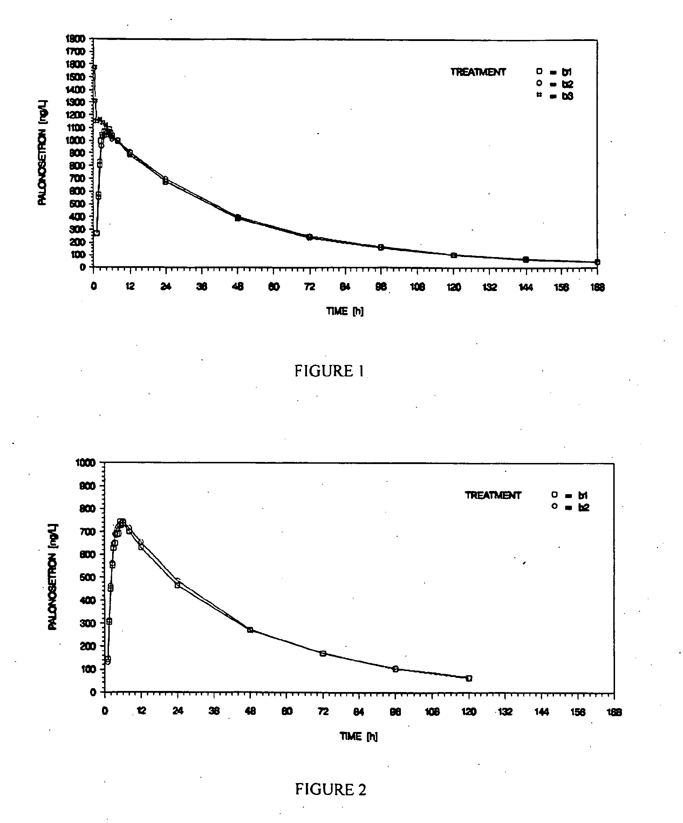

Pharmacokinetics are also reported in figure 1, wherein b1 represents treatment by Formulation A, b2 represents treatment by Formulation B, and b3 represented treatment by Aloxi i.v. The figure reports arithmetic mean plasma concentrations of palonosetron (ng/ml) versus time (H) on a linar scale (n=33).

### Example 13 - Boequivalence of 50 mg. clinical and commercial gel cap formulations

A bioequivalence study was undertaken to evaluate single oral doses of two formulations (Formulation A and Formulation B) of palonosetron 0.50 mg. Soft gel capsules in healthy male and female subjects. The study was a two treatments, two periods, two sequences, open label, randomized cross-over study.

Pharmacokinetic results are reported in Table 8.

**Table 8**

| **Pharmacokinetic parameter Palonosetron [unit]** | | **0.50 mg Palonosetron oral administration (formulation A) N=36** | **0.50 mg Palonosetron oral administration (formulation B) N=36** |
|---|---|---|---|
| AUC₀₋ₜ [ng-h/L] | Mean (SD) | 34076 (9874) | 35106(11012) |
| | Geo. Mean (Geo. SD) | 32766 (1.33) | 33530 (1.36) |
| | Median | 33641 | 34981 |
| | Minimum-Maximum | 20085-60189 | 19003-72136 |
| AUC₀₋ₓ [ng·h/L] | Mean (SD) | 37099 (10141) | 38176 (11698) |
| | Geo. Mean (Geo. SD) | 35834 (1.30) | 36555 (1.35) |
| | Median | 36859 | 37627 |
| | Minimum-Maximum | 22439-62727 | 21240-77635 |
| Cₘₐₓ [ng/L] | Mean (SD) | 785.241 (182.437) | 810.176 (165.985) |
| | Geo. Mean (Geo. SD) | 765.702 (1.25) | 793.900 (1.23) |
| | Median | 750.344 | 816.457 |
| | Minimum-Maximum | 463.862-1322.774 | 537.047-1258.878 |
| tₘₐₓ [h] | Median | 5.500 | 5.000 |
| | Minimum-Maximum | 2.000 - 8.000 | 2.000 - 8.000 |

Pharmacokinetic parameters are also reported in figure 2, wherein b1 represents clinical formulation A. and b2 represents commercial formulation B. The figure reports arithmetic mean plasma concentrations of palonosetron (ng/ml) versus time (H) on a linar scale (n=33).

## Claims

1. A soft gelatin capsule for oral administration comprising:
a) a soft gelatin outer shell having an oxygen permeability of less than about 1.0 x 10⁻³ ml·cm/(cm²·24 hr. atm); and
b) a lipophilic liquid inner fill composition comprising:
i) greater than about 50 wt.% of one or more lipophilic components;
ii) from about 1 to about 20 wt.% of water miscibilized or homogenized in said one or more lipophilic components;
iii) from about 0.05 to about 2.0 mg of palonosetron as palonosetron hydrochloride solubilized or dispersed in said water; and
iv) a surfactant,
wherein said capsule exhibits pharmacokinetics when orally ingested in a fasted state that are bioequivalent to a formulation having greater than 95% absolute bioavailability, wherein bioequivalence is established by a 90% confidence interval for AUC which is between 80% and 125%.

2. The capsule of claim 1 wherein said inner fill composition comprises:
a) from 0.5 to 1.0 mg of palonosetron as palonosetron hydrochloride; and .
b) a solubilizing effective amount of a liquid comprising a lipophilic excipient and water.

3. The soft gelatin capsule of claim 1 comprising glycerin in said outer shell and said inner fill composition.

4. The soft gelatin capsule of claim 1, wherein:
a) said inner fill composition further comprises an antioxidant or a reducing agent;
b) said palonosetron comprises less than about 1 wt.% of (3S)-3-[(3aS)-1-oxo-2,3,3a,4,5,6-hexahydro-1*H*-benzo[*de*]isoquinoline-2-yl]-1-azo-niabicyclo[2.2.2]octan-1-olate.

5. The capsule of claim 1 exhibiting pharmacokinetics when orally ingested in a fasted state that are bioequivalent to a formulation having greater than 95% absolute bioavailability, and a Cₘₐₓ of from 800 to 820 ng/L, wherein bioequivalence is established by:
a) a 90% confidence interval for AUC which is between 80% and 125%, and
b) a 90% confidence interval for Cₘₐₓ which is between 80% and 125%.

6. The capsule of claim 1 wherein said inner fill composition comprises oxygen in an amount that mediates no more than about 3.0 wt.% oxidative degradation when said dosage form is stored for three months at 40°C and 75% RH.

7. The capsule of claim 1 wherein no less than about 75% of said palonosetron or pharmaceutically acceptable salt thereof dissolves in 45 minutes when tested in a type II paddle dissolution apparatus according to the U.S. Pharmacopeia, at 75 rpm and 37°C, in 500 ml. of 0.01 N HCI.

8. The capsule of claim 1 wherein no less than about 75% of said palonosetron or pharmaceutically acceptable salt thereof dissolves in 30 minutes when tested in a type II paddle dissolution apparatus according to the U.S. Pharmacopeia, at 75 rpm and 37°C, in 500 ml. of 0.01 N HCI.

9. The capsule of claim 1 wherein said shell has an oxygen permeability of less than about 1.0 x 10⁻⁴ ml·cm/(cm²·24 hr.atm).

10. The capsule of claim 1 wherein said inner fill comprises from 0.5 to 4 wt.% of a surfactant.

11. The capsule of claim 1 wherein said palonosetron or pharmaceutically acceptable salt thereof comprises (3S)-3-[(3aS)-1-oxo-2,3,3a,4,5,6-hexahydro-1*H*-benzo[*de*]isoquinoline-2-yl]-1-azoniabicyclo[2.2.2]octan-1-olate of a pharmaceutically acceptable salt thereof in an amount of less than 1.0 wt.% based on the weight of said palonosetron, and wherein said inner fill composition comprises oxygen in an amount that mediates no more than about 3.0 wt.% oxidative degradation of said palonosetron or pharmaceutically acceptable salt thereof when said dosage form is stored three months or greater at 40°C and 75% RH.

12. The capsule of claim 11 exhibiting pharmacokinetics when orally ingested in a fasted state that are bioequivalent to a formulation having greater than 90% absolute bioavailability wherein bioequivalence is established by a 90% confidence interval for AUC which is between 80% and 125%.

13. The capsule of claim 11 wherein said inner fill composition comprises from about 1 wt.% to about 20 wt.% water.

14. The capsule of claim 11 exhibiting pharmacokinetics when orally ingested in a fasted state that are bioequivalent to a formulation having greater than 95% absolute bioavailability, and a Cₘₐₓ of from 800 to 820 ng/L, wherein bioequivalence is established by:
a) a 90% confidence interval for AUC which is between 80% and 125%, and
b) a 90% confidence interval for Cₘₐₓ which is between 80% and 125%.

15. The capsule of claim 11 wherein no less than about 75% of said palonosetron or pharmaceutically acceptable salt thereof dissolves in 45 minutes when tested in a type II paddle dissolution apparatus according to the U.S. Pharmacopeia, at 75 rpm and 37°C, in 500 ml of 0.01 N HCl.

16. The capsule of claim 11 wherein said shell has an oxygen permeability of less than about 1.0 x 10⁻⁴ ml·cm/(cm²·24 hr.atm).

17. The capsule of claim 11 wherein:
a) said inner fill composition comprises glycerin; and
b) said shell comprises glycerin.

18. The capsule of claim 11 wherein said shell comprises gelatin, cellulose, starch or HPMC.

19. A method of optimizing the bioavailability and stability of palonosetron in a palonosetron gelatin capsule comprising:
a) providing a soft gelatin outer shell having an oxygen permeability of less than about 1.0 x 10⁻³ ml·cm/(cm²·24 hr.atm); and
b) preparing a fill composition by steps comprising:
i) providing from about 0.05 to about 2.0 mg of palonosetron as palonosetron hydrochloride wherein said palonosetron comprises (35)-3-[(3aS)-1-oxo-2,3,3a,4;5,6-hexahydro-1*H*-benzo[*de*]isoquinoline-2-yl]-1-azoniabicyclo[2.2.2]octan-1-olate in an amount of less than 3.0 wt.%;
ii) dissolving or dispersing said palonosetron in water to form an aqueous premix;
iii) mixing said aqueous premix with one or more lipophilic excipients, at a weight ratio of aqueous premix to lipophilic excipients of less than 30:70, to form a miscible or homogenous lipophilic fill composition;
iv) mixing a surfactant with said water, said aqueous premix, or said fill composition; and
v) balancing the quantities of surfactant and water in said fill composition to facilitate the bioavailability of palonosetron from said gelatin capsule when orally ingested, and to minimize the degree of palonosetron degradation; and
c) filling said outer shell with said fill composition.

20. The method of claim 19 wherein said fill composition comprises from about 0.1 to about 10.0 wt.% surfactant, and from about 0.1 to about 20 wt.% water.

21. The method of claim 19 wherein said fill composition comprises from about 0.5 to about 4 wt.% surfactant, and from about 1 to about 10 wt.% water.

22. The method of claim 19 wherein said outer shell further comprises glycerin, further comprising mixing said aqueous premix with glycerin, before or after the formation of said lipophilic fill composition.

23. A method of making a batch of palonosetron dosage forms having reduced quantities of impurities and oxygen mediated degradation products comprising:
a) mixing palonosetron hydrochloride and one or more Pharmaceutical acceptable excipients to form a mixture according to claim 1;
b) processing said mixture into a plurality of final dosage forms according to claim 1; and
c) testing one or more of said final dosage forms for one or more palonosetron related compounds selected from (3S)-3-[(3a5)-1-oxo-2,3,3a,4,5,6-hexahydro-1*H*-benzo[*de*]isoquinoline-2-yl]-1-azonia-bicyclo[2.2.2]octan-1-olate, 2-[(3S)-1-Azabicyclo[2.2.2]oct-3-yl]-2,4,5,6-tetrahydro-1*H*-benzo[*de*]isoquinoline-1-one hydrochloride, and (3aR)-2-[(S)-1-Azabicyclo[2.2.2]oct-3-yl]-2,3,3a,4,5,6-hexahydro-1-oxo-1*H*-benz[*de*]isoquinoline hydrochloride, or the hydrochloride salt thereof.

24. The method of claim 23 comprising testing for (3S)-3-[(3aS)-1-oxo-2,3,3a,4,5,6-hexahydro-1*H*-benzo[*de*]isoquinoline-2-yl]-1-azoniabicyclo-[2.2.2]octan-1-olate or the hydrochloride salt thereof.

25. The method of claim 23 comprising testing for 2-[(3S)-1-Azabicyclo[2.2.2]oct-3-yl]-2,4,5,6-tetrahydro-1*H*-benzo[*de*]isoquinoline-1-one hydrochloride or the hydrochloride salt thereof.

26. The method of claim 23 comprising testing for (3aR)-2-[(S)-1-Azabicyclo[2.2.2]oct-3-yl]-2,3,3a,4,5,6-hexahydro-1-oxo-1*H*-benz[*de*]-isoquinoline hydrochloride or the hydrochloride salt thereof.

27. The method of claim 23, further comprising testing said palonosetron hydrochloride or said final dosage form for one or more compounds selected from (3S)-3-(1-oxo-2,4,5,6-tetrahydro-1*H*-benzo[*de*] isoquinoline-2-yl)-1-azoniabicyclo[2.2.2]octan-1-olate, (3aR)-2-[(R)-1-Azabicyclo-[2.2.2]oct-3yl]-2,3,3a,4,5,6-hexahydro-1-oxo-1*H*-benz[*de*]isoquinoline hydrochloride, (3aS)-2-[(R)-1-Azabicyclo[2.2.2]oct-3yl]-2,3,3a,4,5,6-hexahydro-1-oxo-1*H*-benz[*de*]isoquinoline hydrochloride; or (3aS)-2-[(S)-1-Azabicyclo[2.2.2]oct-3yl]-2,3,3a,4,5,6-hexahydro-1-oxo-1*H-*benz[*de*]isoquinoline hydrochloride, or the hydrochloride salt thereof.

## Patentansprüche

1. Weichgelatinekapsel für die orale Verabreichung mit:
a) einer äußeren Hülle aus Weichgelatine mit einer Sauerstoffdurchlässigkeit von weniger als etwa 1,0 x 10⁻³ ml·cm/(cm²·24 h·atm) und
b) einer lipophilen flüssigen inneren Füllzusammensetzung enthaltend:
i) mehr als etwa 50 Gew.-% einer oder mehrerer lipophiler Komponenten;
ii) etwa 1 bis etwa 20 Gew.-% Wasser, das mit der einen oder den mehreren lipophilen Komponenten vermischt oder homogenisiert ist;
iii) etwa 0,05 bis etwa 2,0 mg Palonosetron als Palonosetronhydrochlorid, das in dem Wasser solubilisiert oder dispergiert ist und
iv) ein Tensid,
wobei die Kapsel pharmakokinetische Eigenschaften aufweist, wenn sie oral im Fastenzustand eingenommen wird, die bioäquivalent sind einer Formulierung mit mehr als 95% absoluter biologischer Verfügbarkeit, wobei die Bioäquivalenz festgestellt wird durch ein 90% Konfidenzintervall für AUC, das zwischen 80% und 125% liegt.

2. Kapsel nach Anspruch 1, wobei die Zusammensetzung der inneren Füllung aufweist:
a) 0,5 bis 1,0 mg Palonosetron als Palonosetronhydrochlorid und
b) eine solubilisierend wirksame Menge einer Flüssigkeit, die einen lipophilen Hilfsstoff und Wasser enthält.

3. Weichgelatinekapsel nach Anspruch 1 enthaltend Glycerin in der äußeren Hülle und der Zusammensetzung der inneren Füllung.

4. Weichgelatinekapsel nach Anspruch 1, wobei
a) die Zusammensetzung der inneren Füllung weiterhin ein Antioxidans oder ein Reduktionsmittel enthält;
b) das Palonosetron weniger als etwa 1 Gew.-% (3S)-3-[(3aS)-1-Oxo-2,3,3a,4,5,6-hexahydro-1*H*-benzo[*de*]isochinolin-2-yl]-1-azoniabicyclo[2.2.2]octan-1-olat enthält.

5. Kapsel nach Anspruch 1, die pharmakokinetische Eigenschaften aufweist, wenn sie oral in Fastenzustand eingenommen wird, die bioäquivalent sind einer Formulierung mit mehr als 95% absoluter biologischer Verfügbarkeit und einer Cₘₐₓ von 800 bis 820 ng/l,
wobei die Bioäquivalenz festgestellt wird durch:
a) ein 90% Konfidenzintervall für AUC, das zwischen 80% und 125% liegt und
b) ein 90% Konfidenzintervall für Cₘₐₓ, das zwischen 80% und 125% liegt.

6. Kapsel nach Anspruch 1, wobei die Zusammensetzung der inneren Füllung Sauerstoff in einer Menge enthält, die nicht mehr als etwa 3,0 Gew.-% oxidativen Abbau vermittelt, wenn die Dosierungsform drei Monate bei 40°C und 75% RH gelagert wird.

7. Kapsel nach Anspruch 1, wobei nicht weniger als etwa 75% des Palonosetrons oder des pharmazeutisch annehmbaren Salzes davon sich in 45 Minuten lösen, wenn sie in einer Typ-II-Paddle-Auflösungsvorrichtung getestet werden gemäß US-Pharmakopöe bei 75 Upm und 37°C in 500 ml 0,01 n HCI.

8. Kapsel nach Anspruch 1, wobei nicht weniger als etwa 75% des Palonosetrons oder eines pharmazeutisch annehmbaren Salzes davon sich in 30 Minuten lösen, wenn sie in einer Typ-II-Paddle-Auflösungsvorrichtung getestet werden gemäß US-Pharmakopöe bei 75 Upm und 37°C in 500 ml 0,01 n HCI.

9. Kapsel nach Anspruch 1, wobei die Hülle eine Sauerstoffdurchlässigkeit von weniger als etwa 1,0 x 10⁻⁴ ml·cm/(cm²·24 h·atm) hat.

10. Kapsel nach Anspruch 1, wobei die innere Füllung 0,5 bis 4 Gew.-% eines Tensids enthält.

11. Kapsel nach Anspruch 1, wobei das Palonosetron oder das pharmazeutisch annehmbare Salz davon (35)-3-[(3aS)-1-Oxo-2,3,3a,4,5,6-hexahydro-1*H*-benzo[*de*]isochinolin-2-yl]-1-azoniabicyclo[2.2.2]octan-1-olat oder ein pharmazeutisch annehmbares Salz davon in einer Menge von weniger als 1,0 Gew.-% bezogen auf das Gewicht des Palonosetrons enthält und wobei die Zusammensetzung der inneren Füllung Sauerstoff in einer Menge enthält, die nicht mehr als etwa 3,0 Gew.-% oxidativen Abbau des Palonosetrons oder des pharmazeutisch annehmbaren Salzes davon vermittelt, wenn die Dosierungsform drei Monate oder länger bei 40°C und 75% RH gelagert wird.

12. Kapsel nach Anspruch 11, die pharmakokinetische Eigenschaften aufweist, wenn sie oral in Fastenzustand eingenommen wird, die bioäquivalent sind einer Formulierung mit mehr als 90% absoluter biologischer Verfügbarkeit, wobei die Bioäquivalenz festgestellt wird durch ein 90% Konfidenzintervall für AUC, das zwischen 80% und 125% liegt.

13. Kapsel nach Anspruch 11, wobei die Zusammensetzung der inneren Füllung etwa 1 Gew.-% bis etwa 20 Gew.-% Wasser enthält.

14. Kapsel nach Anspruch 11, die pharmakokinetische Eigenschaften aufweist, wenn sie oral in Fastenzustand eingenommen wird, die bioäquivalent sind einer Formulierung mit mehr als 95% absoluter biologischer Verfügbarkeit und einer Cₘₐₓ von 800 bis 820 ng/l,
wobei die Bioäquivalenz festgestellt wird durch:
a) ein 90% Konfidenzintervall für AUC, das zwischen 80% und 125% liegt und
b) ein 90% Konfidenzintervall für Cₘₐₓ, das zwischen 80% und 125% liegt.

15. Kapsel nach Anspruch 11, wobei nicht weniger als etwa 75% des Palonosetrons oder des pharmazeutisch annehmbaren Salzes davon sich in 45 Minuten lösen, wenn in einer Typ-II-Paddle-Auflösungsvorrichtung getestet wird gemäß der US-Pharmakopöe bei 75 Upm und 37°C in 500 ml 0,01 n HCl.

16. Kapsel nach Anspruch 11, wobei die Hülle eine Sauerstoffdurchlässigkeit von weniger als etwa 1,0 x 10⁻⁴ ml·cm/(cm²·24 h·atm) hat.

17. Kapsel nach Anspruch 11, wobei:
a) die Zusammensetzung der inneren Füllung Glycerin enthält und
b) die Hülle Glycerin enthält.

18. Kapsel nach Anspruch 11, wobei die Hülle Gelatine, Cellulose, Stärke oder HPMC enthält.

19. Verfahren zur Optimierung der biologischen Verfügbarkeit und Stabilität von Palonosetron in einer Palonosetrongelatinekapsel umfassend:
a) dass eine äußere Hülle aus Weichgelatine mit einer Sauerstoffdurchlässigkeit von weniger als etwa 1,0 x 10⁻³ ml·cm/(cm²·24 h·atm) bereitgestellt wird und
b) eine Füllzusammensetzung hergestellt wird mit den folgenden Stufen:
i) dass etwa 0,05 bis etwa 2,0 mg Palonosetron als Palonosetronhydrochlorid bereitgestellt werden, wobei das Palonosetron (3S)-3-[(3aS)-1-Oxo-2,3,3a,4,5,6-hexahydro-1*H*-benzo[*de*]isochinolin-2-yl]-1-azoniabicyclo[2.2.2]octan-1-olat in einer Menge von weniger als 3,0 Gew.-% enthält;
ii) dass das Palonosetron in Wasser gelöst oder dispergiert wird, um eine wässrige Vormischung zu bilden;
iii) dass die wässrige Vormischung mit einem oder mehreren lipophilen Hilfsstoffen vermischt wird mit einem Gewichtsverhältnis von wässriger Vormischung zu lipophilen Hilfsstoffen von weniger als 30:70, um eine mischbare oder homogene lipophile Füllzusammensetzung zu bilden;
iv) ein Tensid mit Wasser, der wässrigen Vormischung oder der Füllzusammensetzung vermischt wird und
v) die Mengen an Tensid und Wasser so eingestellt werden, dass die Füllzusammensetzung die biologische Verfügbarkeit von Palonosetron aus der Gelatinekapsel erleichtert, wenn diese oral eingenommen wird und der Zersetzungsgrad von Palonosetron minimiert wird und
c) die äußere Hülle mit der Füllzusammensetzung gefüllt wird.

20. Verfahren nach Anspruch 19, wobei die Füllzusammensetzung etwa 0,1 bis etwa 10,0 Gew.-% Tensid und etwa 0,1 bis etwa 20 Gew.-% Wasser enthält.

21. Verfahren nach Anspruch 19, wobei die Füllzusammensetzung etwa 0,5 bis etwa 4 Gew.-% Tensid und etwa 1 bis etwa 10 Gew.-% Wasser enthält.

22. Verfahren nach Anspruch 19, wobei die äußere Hülle weiterhin Glycerin enthält, wobei weiterhin umfasst ist, dass die wässrige Vormischung mit Glycerin vermischt wird, vor oder nach der Bildung der lipophilen Füllzusammensetzung.

23. Verfahren zur Herstellung einer Charge von Palonosetrondosierungsformen mit verminderten Mengen an Verunreinigung und durch Sauerstoff vermittelten Abbauprodukten umfassend:
a) dass Palonosetronhydrochlorid und ein oder mehrere pharmazeutisch annehmbare Hilfsstoffe vermischt werden, um eine Mischung gemäß Anspruch 1 zu bilden;
b) die Mischung zu einer Vielzahl von fertigen Dosierungsformen gemäß Anspruch 1 verarbeitet wird und
c) ein oder mehrere der fertigen Dosierungsformen auf eine oder mehrere mit Palonosetron verwandte Verbindungen ausgewählt aus (3S)-3-[(3aS)-1-Oxo-2,3,3a,4,5,6-hexahydro-1*H*-benzo[*de*]-isochinolin-2-yl]-1-azoniabicyclo[2.2.2]octan-1-olat, 2-[(3S)-1-Azabicyclo[2.2.2]oct-3-yl]-2,4,5,6-tetrahydro-1*H*-benzo[*de*]isochinolin-1-onhydrochlorid und (3aR)-2-[(S)-1-Azabicyclo[2.2.2]oct-3-yl]-2,3,3a,4,5,6-hexahydro-1-oxo-1*H*-benz[*de*]isochinolinhydrochlorid oder ein Hydrochloridsalz davon getestet werden.

24. Verfahren nach Anspruch 23 umfassend, dass auf (3S)-3-[(3aS)-1-Oxo-2,3,3a,4,5,6-hexahydro-1*H*-benzo[*de*]isochinolin-2-yl]-1-azoniabicyclo[2.2.2]octan-1-olat oder das Hydrochloridsalz davon getestet wird.

25. Verfahren nach Anspruch 23 umfassend, dass auf 2-[(3S)-1-Azabicyclo[2.2.2]oct-3-yl]-2,4,5,6-tetrahydro-1*H*-benzo[*de*]isochinolin-1-onhydrochlorid oder das Hydrochlorid davon getestet wird.

26. Verfahren nach Anspruch 23 umfassend, dass auf (3aR)-2-[(S)-1-Azabicyclo[2.2.2]oct-3-yl]-2,3,3a,4,5,6-hexahydro-1-oxo-1*H*-benz[*de*]isochinolinhydrochlorid oder das Hydrochlorid davon getestet wird.

27. Verfahren nach Anspruch 23 weiter umfassend, dass Palonosetronhydrochlorid oder die fertige Dosierungsform auf eine oder mehrere Verbindungen getestet werden ausgewählt aus (3S)-3-(1-Oxo-2,4,5,6-tetrahydro-1*H*-benzo[*de*]isochinolin-2-yl)-1-azoniabicyclo[2.2.2]octan-1-olat, (3aR)-2-[(R)-1-Azabicyclo-[2.2.2]oct-3-yl]-2,3,3a,4,5,6-hexahydro-1-oxo-1*H*-benz[*de*]isochinolinhydrochlorid, (3aS)-2-[(R)-1-Azabicyclo[2.2.2]oct-3-yl]-2,3,3a,4,5,6-hexahydro-1-oxo-1*H*-benz[*de*]isochinolinhydrochlorid oder (3aS)-2-[(S)-1-Azabicyclo[2.2.2]oct-3-yl]-2,3,3a,4,5,6-hexahydro-1-oxo-1*H*-benz[*de*]isochinolinhydrochlorid oder das Hydrochloridsalz davon.

## Revendications

1. Capsule en gélatine molle destinée à une administration orale comprenant :
a) une enveloppe extérieure en gélatine molle présentant une perméabilité à l'oxygène inférieure à 1,0 × 10⁻³ ml·cm/(cm²·24 h·atm) environ, et
b) une composition de remplissage intérieur de liquide lipophile comprenant :
i) une proportion supérieure à environ 50 % en poids d'un ou de plusieurs composants lipophiles,
ii) une proportion d'environ 1 à environ 20 % en poids d'eau miscible ou homogénéisée dans lesdits un ou plusieurs composants lipophiles,
iii) une masse d'environ 0,05 à environ 2,0 mg de palonosétron sous la forme de chlorhydrate de palonosétron solubilisé ou dispersé dans ladite eau, et
iv) un tensioactif,
où ladite capsule présente une pharmacocinétique lorsqu'elle est ingérée oralement à jeun qui est bioéquivalente à une formulation présentant une biodisponibilité absolue supérieure à 95 %, où une bioéquivalence est établie par un intervalle de confiance de 90 % pour une surface sous la courbe (AUC) qui est comprise entre 80 % et 125 %.

2. Capsule selon la revendication 1, dans laquelle ladite composition de remplissage intérieur comprend :
a) une masse de 0,5 à 1,0 mg de palonosétron sous la forme de chlorhydrate de palonosétron, et
b) une quantité effective pour solubilisation d'un liquide comprenant un excipient lipophile et de l'eau.

3. Capsule en gélatine molle selon la revendication 1, comprenant de la glycérine dans ladite enveloppe extérieure et dans ladite composition de remplissage intérieur.

4. Capsule en gélatine molle selon la revendication 1, dans laquelle :
a) ladite composition de remplissage intérieur comprend en outre un antioxydant ou un agent de réduction,
b) ledit palonosétron comprend moins de 1 % en poids environ de (3S)-3-[(3aS)-1-oxo-2,3,3a,4,5,6-hexahydro-1*H*-benzo[*de*]isoquinoline-2-yl]-1-azoniabicyclo[2.2.2]octan-1-olate.

5. Capsule selon la revendication 1, présentant une pharmacocinétique lorsqu'elle est ingérée oralement à jeun qui est bioéquivalente à une formulation présentant une biodisponibilité absolue supérieure à 95 % et un Cₘₐₓ de 800 à 820 ng/l, où la bioéquivalence est établie par :
a) un intervalle de confiance de 90 % pour une surface sous la courbe (AUC) qui est comprise entre 80 % et 125 %, et
b) un intervalle de confiance de 90 % pour un Cₘₐₓ qui est compris entre 80 % et 125%.

6. Capsule selon la revendication 1, dans laquelle ladite composition de remplissage intérieur comprend de l'oxygène suivant une quantité qui n'engendre pas plus d'environ 3,0 % en poids de dégradation oxydative lorsque ladite forme de dosage est stockée pendant trois mois à 40 °C et à 75 % d'humidité relative.

7. Capsule selon la revendication 1, dans laquelle pas moins d'environ 75 % dudit palonosétron ou d'un sel pharmaceutiquement acceptable de celui-ci se dissolvent en 45 minutes lorsqu'il est testé dans un appareil de dissolution à pales de type II conformément à la Pharmacopée américaine, à 75 tr/min et à 37 °C, dans 500 ml de HCl à 0,01 N.

8. Capsule selon la revendication 1, dans laquelle pas moins d'environ 75 % dudit palonosétron ou d'un sel pharmaceutiquement acceptable de celui-ci se dissolvent en 45 minutes lorsqu'il est testé dans un appareil de dissolution à pales de type II conformément à la Pharmacopée américaine, à 75 tr/min et à 37 °C, dans 500 ml de HCI à 0,01 N.

9. Capsule selon la revendication 1, dans laquelle ladite enveloppe présente une perméabilité à l'oxygène de moins de 1,0 × 10⁻⁴ ml·cm/(cm²·24 h·atm) environ.

10. Capsule selon la revendication 1, dans laquelle ledit remplissage intérieur comprend de 0,5 à 4 % en poids d'un tensioactif.

11. Capsule selon la revendication 1, dans laquelle ledit palonosétron ou sel pharmaceutiquement acceptable de celui-ci comprend du (3S)-3-[(3aS)-1-oxo-2,3,3a,4,5,6-hexahydro-1*H*-benzo[de]isoquinoline-2-yl]-1-azoniabicyclo[2.2.2]octan-1-olate ou un sel pharmaceutiquement acceptable de celui-ci suivant une proportion inférieure à 1,0 % en poids sur la base de la masse dudit palonosétron et dans laquelle ladite composition de remplissage intérieur comprend de l'oxygène suivant une quantité qui n'engendre pas plus d'environ 3,0 % en poids de dégradation oxydative dudit palonosétron ou sel pharmaceutiquement acceptable de celui-ci lorsque ladite forme de dosage est stockée pendant trois mois ou plus à 40 °C et à 75 % d'humidité relative.

12. Capsule selon la revendication 11, présentant une pharmacocinétique lorsqu'elle est ingérée oralement à jeun qui est bioéquivalente à une formulation présentant une biodisponibilité absolue supérieure à 90 %, où la bioéquivalence est établie par un intervalle de confiance de 90 % pour une surface sous la courbe (AUC) qui est comprise entre 80 % et 125 %.

13. Capsule selon la revendication 11, dans laquelle ladite composition de remplissage intérieur comprend d'environ 1 % en poids à environ 20 % en poids d'eau.

14. Capsule selon la revendication 11, présentant une pharmacocinétique lorsqu'elle est ingérée oralement à jeun qui est bioéquivalente à une formulation présentant une biodisponibilité absolue supérieure à 95 % et un Cₘₐₓ de 800 à 820 ng/l, où la bioéquivalence est établie par :
a) un intervalle de confiance de 90 % pour une surface sous la courbe (AUC) qui est comprise entre 80 % et 125 %, et
b) un intervalle de confiance de 90 % pour un Cₘₐₓ qui est compris entre 80 % et 125%.

15. Capsule selon la revendication 11, dans laquelle pas moins d'environ 75 % dudit palonosétron ou sel pharmaceutiquement acceptable de celui-ci se dissolvent en 45 minutes lorsqu'il est testé dans un appareil de dissolution à pales de type II conformément à la Pharmacopée américaine, à 75 tr/min et à 37 °C, dans 500 ml de HCI à 0,01 N.

16. Capsule selon la revendication 11, dans laquelle ladite enveloppe présente une perméabilité à l'oxygène inférieure à 1,0 × 10⁻⁴ ml·cm/(cm²·24 h·atm) environ.

17. Capsule selon la revendication 11, dans laquelle :
a) ladite composition de remplissage intérieur comprend de la glycérine, et
b) ladite enveloppe comprend de la glycérine.

18. Capsule selon la revendication 11, dans laquelle ladite enveloppe comprend de la gélatine, de la cellulose, de l'amidon ou de l'hydroxypropylméthylcellulose (HPMC).

19. Procédé d'optimisation de la biodisponibilité et de la stabilité du palonosétron dans une capsule en gélatine avec palonosétron comprenant les étapes consistant à :
a) fournir une enveloppe extérieure en gélatine molle présentant une perméabilité à l'oxygène inférieure à 1,0 × 10⁻³ ml·cm/(cm²·24 h·atm) environ, et
b) préparer une composition de remplissage par étapes comprenant :
i) la fourniture d'environ 0,05 à environ 2,0 mg de palonosétron sous la forme de chlorhydrate de palonosétron où ledit palonosétron comprend le (3S)-3-[(3aS)-1-oxo-2,3,3a,4,5,6-hexahydro-1*H-*benzo[de]isoquinoline-2-yl]-1-azoniabicyclo[2.2.2]octan-1-olate suivant une proportion inférieure à 3,0 % en poids,
ii) la dissolution ou la dispersion dudit palonosétron dans l'eau pour former un pré-mélange aqueux,
iii) le mélange dudit pré-mélange aqueux avec un ou plusieurs excipients lipophiles, selon un rapport massique du pré-mélange aqueux sur les excipients lipophiles inférieur à 30:70, pour former une composition de remplissage lipophile miscible ou homogène,
iv) le mélange d'un tensioactif avec ladite eau, ledit pré-mélange aqueux ou ladite composition de remplissage, et
v) l'équilibrage des quantités de tensioactif et d'eau dans ladite composition de remplissage pour faciliter la biodisponibilité du palonosétron à partir de ladite capsule en gélatine lorsqu'elle est ingérée oralement et pour réduire au minimum le degré de dégradation du palonosétron, et
c) remplir ladite enveloppe extérieure avec ladite composition de remplissage.

20. Procédé selon la revendication 19, dans lequel ladite composition de remplissage comprend d'environ 0,1 à environ 10,0 % en poids de tensioactif et d'environ 0,1 à environ 20 % en poids d'eau.

21. Procédé selon la revendication 19, dans lequel ladite composition de remplissage comprend d'environ 0,5 à environ 4 % en poids de tensioactif et d'environ 1 à environ 10 % en poids d'eau.

22. Procédé selon la revendication 19, dans lequel ladite enveloppe extérieure comprend en outre de la glycérine, comprenant en outre le mélange dudit pré-mélange aqueux avec la glycérine, avant ou après la formation de ladite composition de remplissage lipophile.

23. Procédé de réalisation d'un lot de formes de dosage avec palonosétron présentant des quantités réduites d'impuretés et de produits de dégradation engendrés par l'oxygène, comprenant les étapes consistant à :
a) mélanger le chlorhydrate de palonosétron et un ou plusieurs excipients pharmaceutiquement acceptables pour former un mélange selon la revendication 1,
b) transformer ledit mélange en une pluralité de formes de dosage finales selon la revendication 1, et
c) tester une ou plusieurs desdites formes de dosage finales en ce qui concerne un ou plusieurs des composés associés au palonosétron sélectionnés à partir du (3S)-3-[(3aS)-1-oxo-2,3,3a,4,5,6-hexahydro-1*H*-benzo[de]isoquinoline-2-yl]-1-azoniabicyclo[2.2.2]octan-1-olate, du chlorhydrate de 2-[(3S)-1-azabicyclo[2.2.2]oct-3-yl]-2,4,5,6-tétrahydro-1*H*-benzo[de]isoquinoline-1-one et du chlorhydrate de (3aR)-2-[(S)-1-azabicyclo[2.2.2]oct-3-yl]-2,3,3a,4,5,6-hexahydro-1-oxo-1*H*-benz[de]isoquinoline ou du sel de chlorhydrate de ceux-ci.

24. Procédé selon la revendication 23, comprenant un test en ce qui concerne le (3S)-3-[(3aS)-1-oxo-2,3,3a,4,5,6-hexahydro-1*H*-benzo[de]isoquinoline-2-yl]-1-azoniabicyclo[2.2.2]octan-1-olate ou le sel de chlorhydrate de celui-ci.

25. Procédé selon la revendication 23, comprenant un test en ce qui concerne le chlorhydrate de 2-[(3S)-1-azabicyclo[2.2.2]oct-3-yl]-2,4,5,6-tétrahydro-1*H-*benzo[de]isoquinoline-1-one ou le sel de chlorhydrate de celui-ci.

26. Procédé selon la revendication 23, comprenant un test en ce qui concerne le chlorhydrate de (3aR)-2-[(S)-1-azabicyclo[2.2.2]oct-3-yl]-2,3,3a,4,5,6-hexahydro-1-oxo-1*H*-benz[de]isoquinoline ou le sel de chlorhydrate de celui-ci.

27. Procédé selon la revendication 23, comprenant en outre un test dudit chlorhydrate de palonosétron ou de ladite forme de dosage finale en ce qui concerne un ou plusieurs composés sélectionnés à partir du (3S)-3-(1-oxo-2,4,5,6-tétrahydro-1*H-*benzo[de]isoquinoline-2-yl)-1-azoniabicyclo[2.2.2]octan-1-olate, du chlorhydrate de (3aR)-2-[(R)-1-azabicyclo[2.2.2]oct-3-yl]-2,3,3a,4,5,6-hexahydro-1-oxo-1*H-*benz[de]isoquinoline, du chlorhydrate de (3aS)-2-[(R)-1-azabicyclo[2.2.2]oct-3-yl]-2,3,3a,4,5,6-hexahydro-1-oxo-1*H*-benz[de]isoquinoline ou du chlorhydrate de (3aS)-2-[(S)-1-azabicyclo[2.2.2]oct-3-yl]-2,3,3a,4,5,6-hexahydro-1-oxo-1*H*-benz[de]isoquinoline, ou du sel de chlorhydrate de ceux-ci.
